# EUROPÄISCHE PATENTSCHRIFT

(11) **EP 3 773 376 B1**
(45) Veröffentlichungstag und Bekanntmachung des Hinweises auf die Patenterteilung: **29.12.2021**
(21) Anmeldenummer: 19720373.0
(22) Anmeldetag: 03.04.2019
(51) Int. Cl.: A61F 9/007, A61B 17/22

(54) **PHACO-EMULSIFIKATIONSHANDSTÜCK**
PHACOEMULSIFICATIONHANDLE
MANCHE POUR LA PHACOÉMULSIFICATION

(30) Priorität: 04.04.2018 WO PCT/EP2018/058600
(43) Veröffentlichungstag der Anmeldung: 17.02.2021
(60) Teilanmeldung: 21158592.2
(73) Patentinhaber: Wefis GmbH, 50996 Köln (DE)
(72) Erfinder: WERNZ, Dietrich, 50996 Köln (DE); WÖRSDÖRFER, Stefan, 50996 Köln (DE)
(74) Vertreter: dompatent von Kreisler Selting Werner - Partnerschaft von Patent- und Rechtsanwälten mbB
(86) Internationale Anmeldenummer: PCT/EP2019/058353
(87) Internationale Veröffentlichungsnummer: WO 2019/193027

(56) Entgegenhaltungen:
- EP-A1- 2 011 458
- WO-A1-89/06515
- WO-A1-2007/070081
- WO-A1-2015/014650
- JP-A- H05 177 169
- JP-A- S60 236 638
- US-A1- 2017 281 216
- US-B2- 7 431 728
- US-B2- 8 475 480

## Beschreibung

Die Erfindung betrifft ein Einmalgebrauch-Phaco-Emulsifikationshandstück.

Einmalgebrauch-Phaco-Emulsifikationshandstücke werden als chirurgische Instrumente in der Augenchirurgie eingesetzt. Beispiele für derartige chirurgische Instrumente sind in WO-A-93/15703-A, WO-A-00/53136-A, EP-A-2 011 458, DE-A-102 09 495 und DE-C-4 008 594 beschrieben.

In WO-A-89/06515 ist eine Vorrichtung für die In-Vivo-Angioplastie beschrieben, die eine Sonotrode aus einer Aluminiumlegierung aufweist, bei der es sich um AI-2024 oder AI-7075 handeln kann.

Aus JP-A-S60 236638 ist ein chirurgisches Instrument für die Ophthalmologie bekannt, bei dem eine Sonotrode eingesetzt wird, die aus einem Duraluminium-Werkstoff besteht.

Medizinische Ultraschallvorrichtungen mit Wellenleitern aus verschiedenen Materialien, wie z.B. Titan, Eisen oder Stahl, sind aus WO-A-2007/070081 bekannt.

Weitere medizinische Ultraschallvorrichtungen sind aus US-B-7 431 728 bekannt.

Ein Phaco-Emulsifikationshandstück dient zum Zertrümmern der Augenlinse und Absaugen von Geweberesten. Zu diesem Zweck ist das Werkzeug als Hohlnadel ausgebildet und wird von einem Vibrationsmechanismus des Handstücks, der als Ultraschallwandler ausgeführt ist, in Hochfrequente lineare Vor- und Zurückbewegungen versetzt. Außen um die Hohlnadel herum wird eine Spülflüssigkeit, beispielsweise eine Emulsionsflüssigkeit zugeführt, die zur Operationsstelle gelangt und von dort zusammen mit herausgelösten Gewebebestandteilen durch das Werkzeug und einen Absaugkanal des Handstücks abgesaugt wird.

Die Kosten für die Herstellung eines Phaco-Emulsifikationshandstücks sind nicht unbeträchtlich und werden im Wesentlichen bestimmt durch den Ultraschallwandler und das Gehäuse. Aufgrund der vergleichsweise hohen Herstellungskosten werden daher Phaco-Emulsifikationshandstücke mehrfach verwendet. Daher müssen die Einzelkomponenten aus vergleichsweise langlebigen Materialien bestehen. Für den Mehrfacheinsatz eines Phaco-Emulsifikationshandstücks ist dessen Sterilisation nach jedem Gebrauch bzw. vor einem weiteren Gebrauch zwingend erforderlich. Auch dies ist mit zusätzlichen Kosten verbunden und überdies problematisch, weshalb bereits darüber nachgedacht wurde, Phaco-Emulsifikationshandstücke für den Einmalgebrauch zu entwickeln. Dies scheiterte bisher allerdings an den weiterhin recht hohen Herstellungskosten.

Aufgabe der Erfindung ist es daher, die Einzelkomponenten eines Phaco-Emulsifikationshandstücks weiter zu vereinfachen, um kostengünstig ein Einmalgebrauch-Instrument herstellen zu können.

Zur Lösung dieser Aufgabe wird mit der Erfindung ein Einmalgebrauch-Phaco-Emulsifikationshandstück vorgeschlagen, das versehen ist mit
- einem Gehäuse,
- einem Ultraschallwandler, der in dem Gehäuse angeordnet ist, und
- einem Werkzeug, dass zwecks Ultraschallanregung mit dem Ultraschallwandler in Wirkverbindung steht,
- wobei der Ultraschallwandler eine Sonotrode, einen Resonator und ein zwischen diesen angeordnetes Piezokeramikelement aufweist und
- wobei die Sonotrode und/oder der Resonator eine Aluminiumlegierung aufweist oder aus einer Aluminiumlegierung besteht, deren Zugfestigkeit größer als 400 N/mm² ist.

Wenn im Folgenden im Zusammenhang mit der Erfindung von "Phaco-Emulsifikationshandstück" gesprochen wird, ist damit ein Einmalgebrauch-Phaco-Emulsifikationshandstück gemeint.

Sinngemäß ist nach der Erfindung vorgesehen, den Ultraschallwandler des Phaco-Emulsifikationshandstücks aus einer Aluminiumlegierung mit einer Mindestzugfestigkeit zu fertigen. Der Ultraschallwandler umfasst die Sonotrode, den Resonator und mindestens ein Piezokeramikelement, das zwischen der Sonotrode und dem Resonator eingespannt gehalten ist. Für die Sonotrode und den Resonator werden nun erfindungsgemäß Aluminiumlegierungen eingesetzt, deren Zugfestigkeit mehr als 400 N/mm² und insbesondere mehr als 450 N/mm² beträgt. Es hat sich überraschenderweise herausgestellt, dass derartige Alumi-niumlegierungen ausreichend stabil sind, um den hochfrequenten Biegewellen, die bei Ansteuerung des Piezokeramikelements entstehen, standzuhalten, und zwar insbesondere für die Dauer einer Operation. Die Kopplung der Sonotrode mit dem Werkzeug ist starr. Ferner ist auch das Werkzeug selbst aus im Wesentlichen starrem oder steifem Material wie Metall und insbesondere z.B. Stahl, Eisen oder Titan ausgebildet, so dass die akustische Energie, d.h. die Schwingungen der Sonotrode im Wesentlichen verlustfrei auf das Werkzeug und durch dieses hindurch übertragen wird/werden.

Nach der Erfindung wird insbesondere als Material für die Sonotrode und den Resonator eine hochfeste Aluminiumlegierung eingesetzt. Beispiele für derartige Legierungen, die sich gemäß zweckmäßiger Ausgestaltungen der Erfindung als geeignet erwiesen haben, sind:
AICu4SiMg
AlCu4Mg1
AlZn4,5Mg1
AlZn5,5MgCu
AlZn5Mg3Cu
AlZn8MgCu.

Im Sinne der Erfindung geeignete Aluminiumlegierungen sind beispielsweise im Merkblatt W2, 11. Auflage, Aluminium-Knetwerkstoffe, herausgegeben vom Gesamtverband der Aluminiumindustrie e.V. (GDA) ISBN 3-937171-00-2, insbesondere Tafel 9, Seiten 54 bis 60, insbesondere Seiten 55 bis 60 angegeben.

In weiterer zweckmäßiger Ausgestaltung der Erfindung ist vorgesehen, dass die Sonotrode einen benachbart zum Resonator angeordneten Mittelabschnitt, in dem die Sonotrode in dem Gehäuse gelagert ist, ein mit dem Resonator gekoppeltes hinteres Ende und ein dem Resonator abgewandtes, gegenüber dem Mittelabschnitt verschlanktes vorderes Ende aufweist, das in Wirkverbindung mit dem Werkzeug steht, und dass sich durch die Sonotrode von deren vorderen Ende bis zu deren mit dem Resonator verbundenen hinteren Ende ein Absaugkanal erstreckt.

Was die Auslegung und das Design eines Ultraschallwandlers für ein Phaco-Emulsifikationshandstück anbelangt, liegen dem Fachmann bekannte Entwurfsregeln vor. Beispielhaft sei verwiesen auf Dr.-Ing. E. G. Lierke, Dr.-Ing. W. Littmann, Dipl.-Ing. D. Simon, Dr.-Ing. T. Hemsel: "Zur Theorie der piezoelektrischen Ultraschallverbundschwinger mit praktischen Schlussfolgerungen für den Entwicklungsingenieur, Universität Paderborn, Fakultät für Maschinenbau, Lehrstuhl für Mechatronik und Dynamik, August 2010.

Wie bereits oben erwähnt, sieht die Erfindung die Verwendung von Aluminiumlegierungen für die Herstellung von der Sonotrode vor. Das schließt aber nicht aus, dass Teile der Sonotrode aus einem anderen metallischen Material als die besagten Aluminiumlegierungen bestehen können.

Die Sonotrode und/oder der Resonator des Ultraschallwandlers ist bzw. sind während des Betriebs erhöhten Temperaturen unterworfen. Bei den bekannten Mehrweg-Phaco-Emulsifikationshandstücken befinden sich die Sonotrode und der Resonator in einem metallischen Innengehäuse, das von einem metallischen Außengehäuse unter Entstehung eines Ringraums umgeben ist. Der Ringraum wird von Spülflüssigkeit durchströmt, die zum Werkstück gelangt, wie bereits oben beschrieben. Diese Spülflüssigkeit kühlt das metallische Innengehäuse und über dieses damit auch die Sonotrode und/oder den Resonator.

Soll ein Einmalgebrauch-Phaco-Emulsifikationshandstück entworfen werden, so sollte kostengünstiges Material für das Gehäuse eingesetzt werden. Wird hier beispielsweise Kunststoff verwendet, so könnte ein Problem darin bestehen, dass das Innenkunststoffgehäuse thermisch bei Weitem nicht mehr so gut leitend ist, wie das bei einem Innengehäuse aus Metall der Fall ist. Damit sollte ein Kunststoff-Innengehäuse Aussparungen aufweisen, die als Kühlöffnungen fungieren und in denen die Sonotrode bzw. der Resonator freiliegt/-liegen. Dann aber gelangt die Spülflüssigkeit in Kontakt mit dem Material des Ultraschallwandlers. Damit sollte dieses Material biokompatibel sein. Es dürfen sich also aus diesem Material bei Kontakt mit der Emulsionsflüssigkeit, bei der es sich beispielsweise um eine Salzlösung handeln kann, keine Reaktionsprodukte bilden, die anschließend von der Spülflüssigkeit bis zum zu behandelnden Gewebe weitergeleitet würden und das Gewebe biochemisch beeinflussen. Daher kann es zweckmäßig sein, diejenigen Komponenten der Sonotrode bzw. des Resonators, die mit Spülflüssigkeit in Kontakt gelangen, aus einen anderen Material, insbesondere einem anderen metallischen Material als demjenigen der erfindungsgemäß eingesetzten Aluminiumlegierungen bestehen, sofern festgestellt wird, dass die verwendete Aluminiumlegierung nicht biokompatibel ist. Hier bietet sich beispielsweise Titan, Eisen, Stahl oder ein anderes biokompatibles Metall bzw. eine entsprechende Metalllegierung an.

Alternativ könnte man zur Lösung des zuvor genannten Problems die Sonotrode und/oder den Resonator mit einer kompatiblen Beschichtung versehen.

In weiterer vorteilhafter Ausgestaltung der Erfindung kann vorgesehen sein, dass der Resonator hohlzylindrisch ausgeführt ist und dass die Sonotrode ein mit dem Resonator mechanisch gekoppeltes, sich durch den Resonator erstreckendes hinteres Ende aufweist, das zur Vorspannung des zwischen dem Resonator und der Sonotrode angeordneten Piezokeramikelements in mechanischem Eingriff, insbesondere in Gewindeeingriff mit dem Resonator steht oder mit diesem klemmend, rastend, verstiftelt oder auf andere Art und Weise verriegelbar ist.

Zum Anschluss des Werkzeugs an die Sonotrode ist es von Vorteil, wenn die Sonotrode an ihrem mit dem Werkzeug in Wirkverbindung stehenden vorderen Ende mit Spiel aus einer Öffnung des Gehäuses herausgeführt ist, wobei auf das Gehäuse an dessen Öffnung ein Haltelement aufsetzbar, insbesondere aufsteck- oder aufschraubbar ist, von dem aus sich eine elastische Hülse erstreckt, die das Werkzeug von außen umgibt. Wie bereits oben beschreiben, wird bei Phaco-Emulsifikationshandstücken auf die Hohlnadel eine elastische Hülse (sogenannter Sleeve) aufgezogen. Diese elastische Hülse befinde sich an einem Haltelement, das die Form einer bei chirurgischen Elementen standardisierten Schraubverbindung aufweist. Das Halteelement wird dabei auf einen Gewindestutzen des Gehäuses aufgeschraubt, der die Öffnung des Gehäuses bildet. Der Außendurchmesser des Gewindestutzens sollte aus den zuvor genannten Gründen normiert sein, um Standardverbindungen verwenden zu können. Das allerdings bedeutet wiederum, dass das aus dem Gewindestutzen herausgeführte bzw. in dem Gewindestutzen angeordnete vordere Ende der Sonotrode ein gewisses Untermaß gegenüber dem Innendurchmesser des Gewindestutzens aufweisen muss, so dass sich zwischen vorderem Ende der Sonotrode und Gewindestutzen ein Ringraum bildet, über den die Spülflüssigkeit aus dem Gehäuse austreten und in den Bereich zwischen Werkzeug und elastischer Hülse gelangen kann. Überraschenderweise hat sich nun gezeigt, dass die erfindungsgemäß vorzusehenden Aluminiumlegierungen auch dann noch ausreichend stabil sind und den beim Betrieb der Sonotrode auftretenden Biegewellen standhalten, obwohl die Wanddicke des vorderen Endes der Sonotrode, das von dem Absaugkanal durchzogen ist, vergleichsweise dünn ist.

Ein gegenüber dem bisherigen Aspekt der Erfindung, nämlich der Verwendung spezieller Aluminiumlegierungen mit einer Mindestzugfestigkeit, separater Gesichtspunkt ist die Möglichkeit, für das Phaco-Emulsifikationshandstück ein Kunststoff-Gehäuse verwenden zu können. Für den Fachmann erschließt sich sogleich, dass es die Verwendung von Kunststoff als Material für das Gehäuse zunächst einmal nicht zwingend erforderlich macht, Sonotrode und/oder Resonator des Ultraschallwandlers aus einer speziellen Aluminiumlegierung bzw. überhaupt aus einem speziellen Material zu fertigen. Insoweit ist also der Vorschlag, Kunststoff als Material für das Gehäuse einzusetzen, als von dem vorherigen Aspekt der Erfindung getrennter Gesichtspunkt zu betrachten.

Gemäß diesem zweiten Aspekt schlägt die Erfindung ein Einmalgebrauch-Phaco-Emulsifikationshandstück vor, das versehen ist mit
- einem Gehäuse,
- einem Ultraschallwandler, der in dem Gehäuse angeordnet ist, und
- einem (insbesondere Metall wie z.B. Stahl, Eisen oder Titan aufweisenden) Werkzeug, dass zwecks Ultraschallanregung mit dem Ultraschallwandler in Wirkverbindung steht,
- wobei der Ultraschallwandler eine Sonotrode, einen Resonator und ein zwischen diesen angeordnetes Piezokeramikelement aufweist und
- wobei das Gehäuse Kunststoff aufweist und/oder aus Kunststoff besteht und
- wobei insbesondere vorgesehen ist, dass das Gehäuse ein zumindest einen Teil der Sonotrode umgebendes Innengehäuseteil und ein Außengehäuseteil aufweist, zwischen denen sich ein Ringraum für die Zuführung und/oder für die Weiterleitung von Spülflüssigkeit zum Werkzeug befindet.

In vorteilhafter Weiterbildung dieses zusätzlichen Aspekts der Erfindung kann vorgesehen sein, dass das Gehäuse ein zumindest einen Teil der Sonotrode umgebendes Innengehäuseteil und ein Außengehäuseteil aufweist, zwischen denen sich ein Ringraum für die Zuführung und/oder für die Weiterleitung von Spülflüssigkeit zum Werkzeug befindet. Wie bereits oben erwähnt, könnte die Kühlung der Sonotrode durch die durch den Ringraum strömende Spülflüssigkeit bei einem Kunststoff-Innengehäuseteil problematisch sein. Dies hängt beispielsweise von der Dicke des Innengehäuseteils ab, wobei anzumerken ist, dass aus Stabilitätsgründen ein gewisses Dickenmaß für das Innengehäuseteil nicht unterschritten werden sollte. Damit ergibt sich möglicherweise die Schwierigkeit, dass das Innengehäuseteil zu dickwandig ist, um aufgrund seiner Umspülung durch die Spülflüssigkeit die Sonotrode noch ausreichend kühlen zu können.

Insoweit von Vorteil ist es, wenn das Innengehäuseteil Kühlöffnungen aufweist, innerhalb derer die Sonotrode und insbesondere der Mittelabschnitt der Sonotrode freiliegt und zur Kühlung mit Spülflüssigkeit in Kontakt gelangt.

Würde man nun die Sonotrode aus einem Aluminiumwerkstoff herstellen, wie es gemäß dem ersten Aspekt der Erfindung vorgeschlagen wird, so stellt sich hier das Problem der möglicherweise nicht mehr gegebenen Biokompatibilität. Daher müsste man durch beispielweise eine entsprechende Beschichtung dafür Sorge tragen, dass der Aluminiumwerkstoff zumindest in denjenigen Bereichen der Sonotrode, in denen diese innerhalb der Kühlöffnungen freiliegt, nicht mit Spülflüssigkeit in Kontakt gelangt.

In weiterer vorteilhafter Ausgestaltung der Erfindung kann vorgesehen sein, dass das Innengehäuseteil eine Austrittsöffnung für das vordere Ende der Sonotrode aufweist und dass das Außengehäuseteil eine mit der Austrittsöffnung des Innengehäuseteils fluchtende und von dieser in Längserstreckung der Sonotrode beabstandete Austrittsöffnung aufweist, in der das vordere Ende der Sonotrode angeordnet ist und/oder über die das vordere Ende der Sonotrode übersteht und auf die ein Halteelement aufsetzbar, insbesondere aufsteck- oder aufschraubbar ist, von dem aus sich eine elastische Hülse erstreckt, die das Werkzeug von außen umgibt. Das Außengehäuseteil steht am vorderen Ende des Phaco-Emulsifikationshandstücks also über das Innengehäuseteil über. Durch die Austrittsöffnung des Innengehäuseteils ragt die Sonotrode aus dem Innengehäuseteil heraus, und zwar insbesondere mit ihrem verschlankten vorderen Ende. Dieses vordere Ende erstreckt sich dann weiter durch das Außengehäuseteil und durch dessen insbesondere als Stutzen ausgebildete Austrittsöffnung bzw. es endet in dieser Austrittsöffnung. Damit schließt sich also an den Ringraum zwischen den beiden Gehäuseteilen ein weiterer Ringraum an, der durch das Außengehäuseteil und die Sonotrode bzw. deren vorderes Ende definiert ist. Auch in diesem Bereich kommt es nun zu einem Kontakt der Spülflüssigkeit mit der Sonotrode. Das Material der Sonotrode sollte also biokompatibel sein. Wenn als Material eine Aluminiumlegierung eingesetzt wird, wie es gemäß dem ersten Aspekt der Erfindung vorgeschlagen wird, und wenn es sich herausstellen sollte, dass dieses Material nicht biokompatibel ist, so müsste man also beispielsweise die Sonotrode in diesem Bereich mit biokompatiblem Material beschichten. Alternativ kann selbstverständlich auch der sich durch den dem Innengehäuseteil vorgelagerten Ringraum erstreckende Teil der Sonotrode aus einem anderen metallischen Material als Aluminium bzw. einer Aluminiumlegierung bestehen. Dann hätte man sozusagen eine Sonotrode, die aus verschiedenen metallischen Materialien besteht. Der besagte Teil der Sonotrode, der nicht aus Aluminium bzw. einer Aluminiumlegierung besteht, kann beispielsweise aus Titan, Eisen oder Stahl oder einem anderen biokompatiblen Metall bestehen, das den beim Betrieb des Ultraschallwandlers auftretenden Biegewellen standhält.

In weiterer zweckmäßiger Ausgestaltung der Erfindung kann vorgesehen sein, dass sich an den Ringraum zwischen dem Innengehäuseteil und dem Außengehäuseteil an der Austrittsöffnung des Innengehäuseteils ein weiterer Ringraum anschließt, der von dem Außengehäuseteil und dem vorderen Ende der Sonotrode gebildet ist und der sich bis in den durch das Spiel zwischen dem vorderen Ende der Sonotrode und der Austrittsöffnung des Außengehäuseteils definierten Zwischenraum erstreckt und von dort in einen Zwischenraum zwischen dem Werkzeug und der elastischen Hülse übergeht.

Wie bereits oben beschrieben, ist der Ultraschallwandler in dem Gehäuse des Phaco-Emulsifikationshandstücks angeordnet und dort gelagert. Zweckmäßig ist es diesbezüglich, wenn die Sonotrode zwei axial beabstandete Aufnahmenuten für jeweils einen Lagerring, insbesondere für einen elastischen Lagerring und vorzugsweise für einen O-Ring aufweist, wobei die beiden Lagerringe zur Lagerung des Ultraschallwandlers von innen an dem Gehäuse und insbesondere an dem Innengehäuseteil anliegen.

Alternativ zur zuvor genannten Lagerung und Zentrierung der Sonotrode in dem Gehäuse bzw. in dem Innengehäuseteil kann vorgesehen sein, dass die Sonotrode an ihrem dem vorderen Ende abgewandten hinteren Ende eine Aufnahmenut für einen Lagerring, insbesondere für einen elastischen Lagerring und vorzugsweise für einen O-Ring, aufweist und dass das vordere Ende der Sonotrode durch nach innen vorstehende Rippen o.dgl. Vorsprünge eines Stutzens des Gehäuses zentriert ist.

Sofern der Innengehäuseteil des erfindungsgemäßen chirurgischen Instruments Kühlöffnungen aufweist, ist es von Vorteil, wenn die Kühlöffnungen in dem zwischen den beiden Lagerringen liegenden Bereich des Gehäuses bzw. des Innengehäuseteils angeordnet sind, wobei die beiden Aufnahmenuten in dem Mittelabschnitt der Sonotrode ausgebildet sind. Die Lagerungsringe dichten also den Bereich beidseitig der Anordnung der Kühlöffnungen ab. Sie dienen damit nicht nur der Lagerung, sondern verhindern auch das Eindringen von Spülflüssigkeit in das Innengehäuseteil.

Zweckmäßig ist es ferner, dass das Gehäuse eine in den Ringraum mündende Einlassöffnung für die Zufuhr von Spülflüssigkeit aufweist.

Vorn Vorteil kann es sein, wenn das Gehäuse an seinem dem vorderen Ende der Sonotrode abgewandten hinteren Ende eine Durchführöffnung für einen Absaugschlauch aufweist, der mit dem sich durch die Sonotrode hindurch erstreckenden Absaugkanal verbunden ist.

In weiterer vorteilhafter Ausgestaltung der Erfindung kann aus dem Gehäuse ein Anschlusskabel herausgeführt werden, dass an das Piezokeramikelement angeschlossen ist.

Die Erfindung wird nachfolgend anhand zweier Ausführungsbeispiele sowie unter Bezugnahme auf die Zeichnung näher erläutert. Im Einzelnen zeigen dabei:
- Fig. 1: einen Längsschnitt durch ein Phaco-Emulsifikationshandstück,
- Fig. 2: einen Querschnitt entlang der Linie II-II der Fig. 1,
- Fig. 3: eine erste Seitenansicht auf das Phaco-Emulsifikationshandstück gemäß Fig. 1 mit teilweise aufgebrochenem und im Schnitt dargestelltem Gehäuse,
- Fig. 4: eine weitere Seitenansicht des Phaco-Emulsifikationshandstücks mit ebenfalls teilweise aufgebrochenem, jedoch in Seitenansicht gezeigtem Innengehäuseteil,
- Fig. 5: eine Seitenansicht des Ultraschallwandlers des Phaco-Emulsifikationshandstück,
- Fig. 6: einen Längsschnitt durch den Ultraschallwandler gemäß Fig. 5,
- Fig. 7: einen Längsschnitt durch ein alternativ ausgebildetes Phaco-Emulsifikationshandstück und
- Fig. 8: einen Querschnitt entlang der Linie VIII-VIII der Fig. 7.

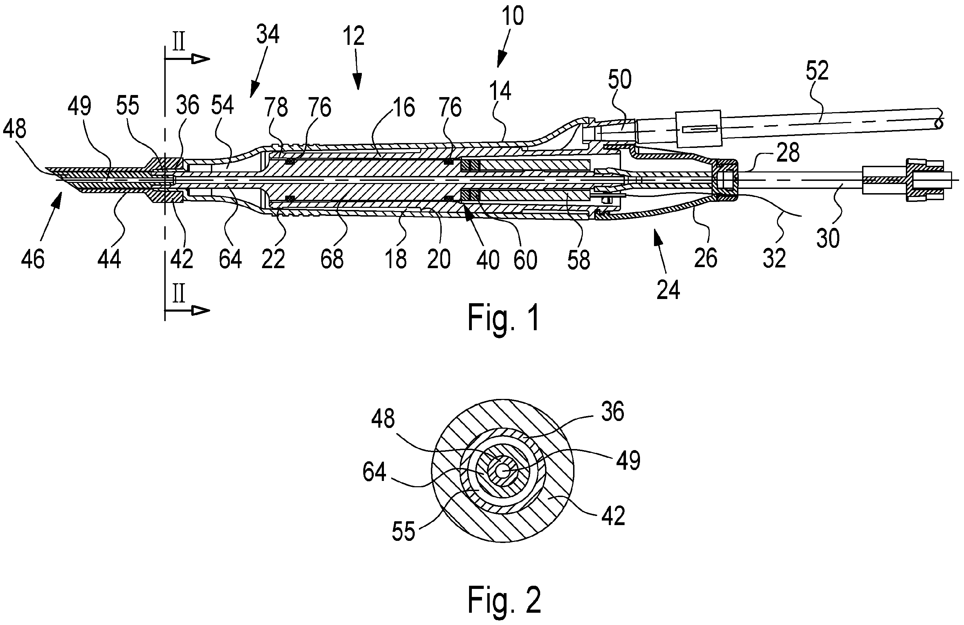

Die Fign. 1 bis 4 zeigen verschiedene Darstellungen eines Phaco-Emulsifikationshandstück 10 gemäß einem ersten Ausführungsbeispiel der Erfindung. Das Handstück 10 weist ein Gehäuse 12 aus Kunststoff auf. Dieses Gehäuse umfasst einen Außengehäuseteil 14 und einen im Außengehäuseteil 14 angeordneten Innengehäuseteil 16. Zwischen beiden Gehäuseteilen 14,16 bildet sich ein erster Ringraum 18, zu dem das Innengehäuseteil 16 durch mehrere Außenvorsprünge 20, 22 zentrisch gelagert ist. Am hinteren Ende 24 des Gehäuses 12 ist dieses durch ein- oder mehrteiliges Deckelteil 26 abgeschlossen, aus dem über eine Durchführöffnung 28 ein Absaugschlauch 30 herausgeführt ist. Ferner ist aus dem Deckelteil 26 ein Anschlusskabel 32 herausgeführt. Auf diese beiden Komponenten wird später noch eingegangen werden.

Wie anhand der Fign. 1 bis 4 zu erkennen ist, überragt das Außengehäuseteil 14 an dem vorderen Ende 34 des Gehäuses 12 das Innengehäuseteil 16. Am vorderen Ende 34 des Gehäuses 12 befindet sich ein Stutzen 36, der eine Austrittsöffnung 38 für einen im Gehäuse 12 angeordneten Ultraschallwandler 40 bildet. Auf den Stutzen 36 ist ein Halteelement 42 aufgeschraubt, an dem sich eine elastische Hülse 44 befindet, die ein Werkzeug 46 in Form einer Hohlnadel 48 (mit Lumen 49) des Handstücks 10 umgibt.

Über eine Einlassöffnung 50, an die ein Spülflüssigkeitsschlauch 52 angeschlossen ist, gelangt Sprühflüssigkeit in den Ringraum 18 des Gehäuses 12, die aus dem Ringraum 18 heraus in einen weiteren Ringraum 54 gelangt und von diesem aus der Austrittsöffnung 38 in den Zwischenraum zwischen Werkzeug 46 und elastischer Hülse 44 gelangt.

Im Innengehäuseteil 16 des Gehäuses 12 befindet sich der bereits oben angesprochene Ultraschallwandler 40 des Phaco-Emulsifikationshandstücks 10.

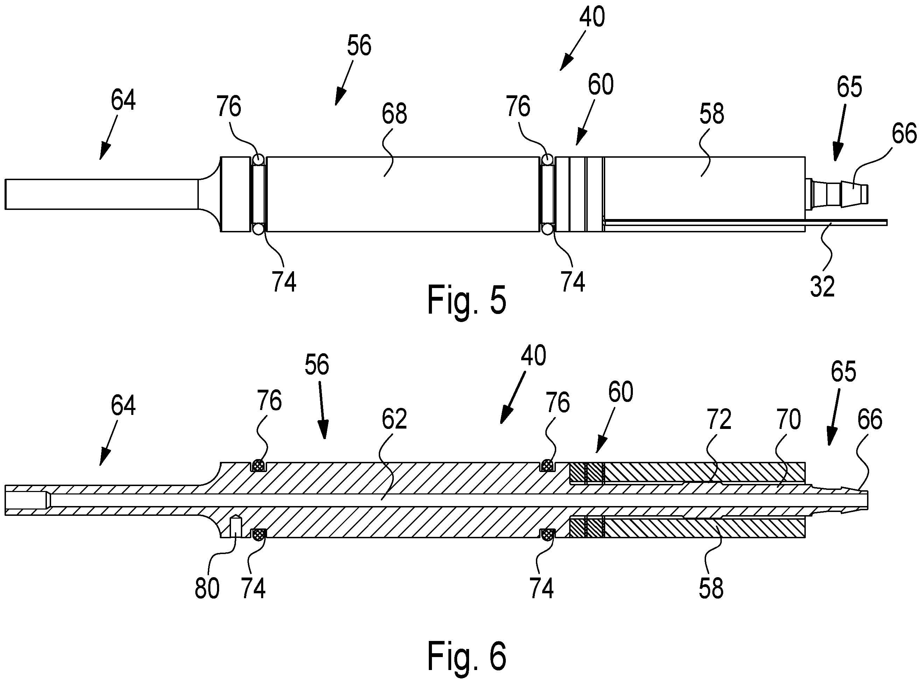

Wie anhand der Fign. 5 und 6 zu erkennen ist, umfasst der Ultraschallwandler 40 eine Sonotrode 56, einen Resonator 58 und mindestens ein Piezokeramikelement 60. An das Piezokeramikelement 60 ist das Anschlusskabel 32 angeschlossen. Durch die Sonotrode 56 hindurch erstreckt sich ein Absaugkanal 62, der an dem vorderen Ende 64 der Sonotrode 56 in Fluidverbindung mit dem Lumen der Hohlnadel 48 steht und an dessen hinterem Ende 65 ein Anschlussstutzen 66 zum Anschluss des Absaugschlauchs 30 vorgesehen ist. Die Sonotrode 56 weist einen Mittelabschnitt 68 auf, gegenüber dem das vordere Ende 64 verschlankt ist. Die Spülflüssigkeit gelangt durch einen Zwischenraum 55 zwischen dem Stutzen 36 und dem vorderen Ende 64 der Sonotrode 56 (siehe Fig. 2), mit dem das Werkzeug 46 verbunden ist, und weiter zwischen das Werkzeug 46 und die elastische Hülse 44, die sich dementsprechend aufweitet, wenn das Werkzeug 46 von Spülflüssigkeit umgeben ist.

Dem vorderen Ende 64 gegenüberliegend weist die Sonotrode 56 ein ebenfalls verschlanktes hinteres Endstück 70 auf, das sich durch den als Hohlzylinder ausgebildeten Resonator 58 erstreckt und mit diesem bei 72 in Gewindeeingriff steht. Auf diese Weise kann das mindestens eine Piezokeramikelement 60 zwischen Resonator 58 und Mittelabschnitt 68 der Sonotrode 56 eingespannt gehalten werden. Der Mittelabschnitt 68 der Sonotrode ist mit zwei Umfangsaufnahmenuten 74 für jeweils einen elastischen Lagerring 76 versehen, über die die Sonotrode 56 und damit der Ultraschallwandler 40 im Innengehäuseteil 16 des Gehäuses 12 gelagert ist.

Die Besonderheit der hier beschriebenen Sonotrode 56 ist in der Auswahl ihres Material zu sehen. Erfindungsgemäß wird eine hochfeste Aluminiumlegierung eingesetzt, deren Zugfestigkeit größer als 400 N/mm² und insbesondere größer als 450 N/mm² ist.

Wir anhand von Fig. 1 zu erkennen ist, umgibt das Innengehäuseteil 16 den Mittelabschnitt 68 der Sonotrode 56 sowie das Piezokeramikelement 60 und den Resonator 58. Im Bereich des Übergangs vom Mittelabschnitt 68 zum verschlankten vorderen Ende 64 ragt die Sonotrode 56 aus dem Innengehäuseteil 16 heraus. An die insoweit am Innengehäuseteil 16 vorgesehene Austrittsöffnung 78 schließt sich der weitere Ringraum 54 an, der definiert ist durch das Außengehäuseteil 14 und das verschlankte vordere Ende 64 der Sonotrode 56.

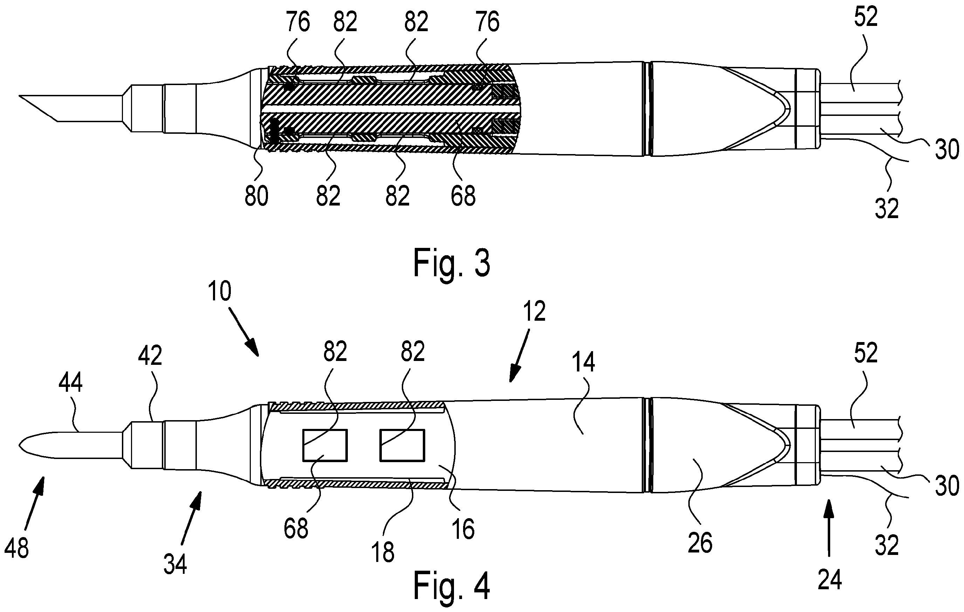

Gemäß einem weiteren Aspekt der Erfindung handelt es sich bei dem Gehäuse 12 um ein Gehäuse aus Kunststoff. Die Sonotrode 56 ist in dem Kunststoffinnengehäuseteil 16 gelagert, und zwar über die beiden Lagerringe 76. Bei 80 ist in den Fign. 3 und 6 angedeutet, dass der Ultraschallwandler 40 im Bereich seiner Sonotrode 56 mit dem Kunststoffinnengehäuseteil 16 verstiftelt ist. Beim Betrieb des Ultraschallwandlers 40 erhitzt sich die Sonotrode 56 merklich, weshalb sie gekühlt werden sollte. Dies erfolgt durch die Spülflüssigkeit, wozu das Kunststoff-Innengehäuseteil 16 im Bereich um den Mittelabschnitt 68 der Sonotrode 56 herum mit mehreren Kühlöffnungen 82 versehen ist (siehe Fign. 3 und 4). Innerhalb dieser Kühlöffnungen 82 liegt die Sonotrode 56 mit ihrem Mittelabschnitt 68 frei, so dass nun die Spulflüssigkeit in diesen Bereichen an der Sonotrode 56 direkt entlangströmen kann, wodurch der Kühleffekt wesentlich effektiver ist als wenn das Kunststoffinnengehäuseteil 16 durchgehend ausgeführt wäre. Dadurch, dass nun die Spülflüssigkeit, die während der Operation mit dem zu behandelnden Gewebe in Kontakt kommt, an der Sonotrode 56 in deren Mittelabschnitt 68 und in deren verschlanktem Ende 64 (nämlich im Ringraum 54) entlangströmt, sollte dafür gesorgt werden, dass das Material der Sonotrode biokompatibel ist. Bei einer Aluminiumlegierung und einer Spülflüssigkeit, die als Kochsalzlösung ausgeführt ist, ist dies möglicherweise nicht in ausreichendem Maße gegeben. Daher sollte die Sonotrode 56 zumindest in den zuvor genannten Bereichen mit einer biokompatiblen Beschichtung versehen sein. Alternativ kann aber auch das gesamte vordere Ende 64 der Sonotrode 56 aus einem biokompatiblen Metall wie beispielsweise Titan bestehen. Dieser Teil der Sonotrode 56 würde dann mit dem restlichen Teil der Sonotrode 56 durch beispielsweise Verschraubung mechanisch verbunden sein.

Wie anhand von Fig. 3 zu erkennen ist, befinden sich die Lagerringe 76 beidseitig der Anordnung der Kühlöffnungen 82 und dichten somit die Sonotrode 56 insbesondere gegenüber dem Resonator 58 und dem Piezokeramikelement 60 ab, so dass in diesen Bereich keine Spülflüssigkeit gelangen kann.

Wie bereits oben erwähnt, handelt es sich bei den Merkmalen der Erfindung und des zuvor beschriebenen Ausführungsbeispiels, soweit sich diese auf das Kunststoff-Gehäuse beziehen, um einen Aspekt der Erfindung, der unabhängig ist von dem anderen Aspekt der Erfindung, nämlich der Verwendung spezieller Alumi-niumlegierungen für den Ultraschallwandler. Die Merkmale des Kunststoffgehäuses und einzelne Varianten davon sind in den nachfolgenden Merkmalsgrup-pen aufgeführt, die in Kombination miteinander treten können, bzw. auch einzeln selbsttätig und in all diesen Fällen die Erfindung und/oder Ausgestaltungen der Erfindung definieren.

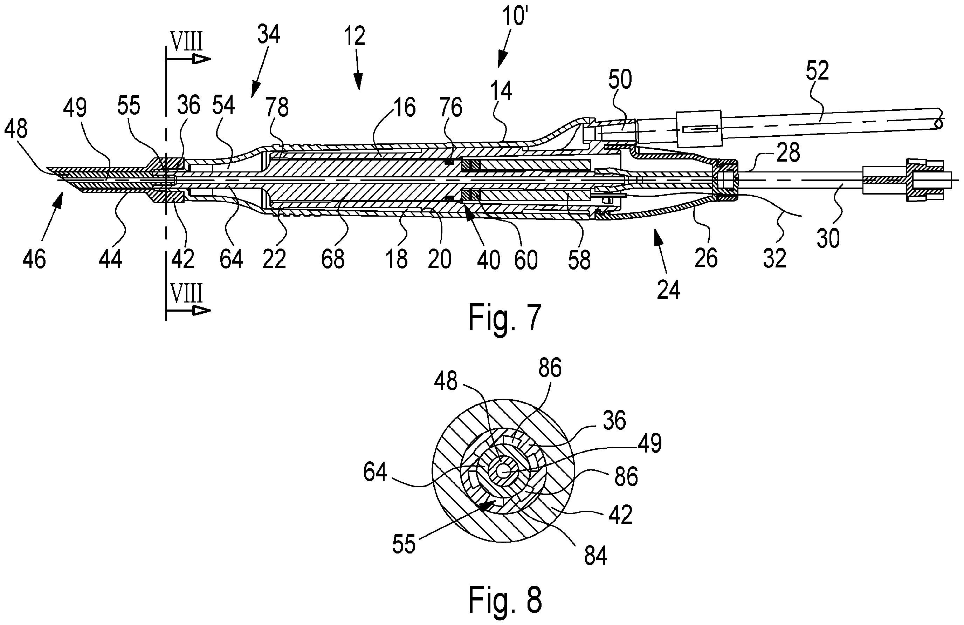

Anhand der Fign. 7 und 8 wird nachfolgend auf eine alternative Ausgestaltung der zentrischen Lagerung der Sonotrode im Gehäuse des Handstücks eingegangen, das in diesen Figuren mit 10' bezeichnet ist. Soweit die einzelnen Elemente des Handstücks 10' der Fign. 7 und 8 konstruktiv oder funktional denjenigen des Handstücks 10 der Fign. 1 bis 6 entsprechen, sind sie in den Fign. 7 und 8 mit den gleichen Bezugszeichen wie in den Fign. 1 bis 6 gekennzeichnet.

Der Unterschied beider Handstücke 10 und 10' ist in der Lagerung der Sonotrode 56 zu sehen. Im Handstück 10' ist die Sonotrode 56 an ihrem hinteren Ende 65 bzw. in ihrem Mittelabschnitt durch den O-Ring 76 gelagert, während ihr vorderes Ende 64 an nach innen vorstehenden Rippen 84 des Stutzens 36 des Außengehäuseteils 14 zentriert ist (siehe Fig. 8). Die Rippen 84 ragen in den Zwischenraum 55 hinein und unterteilen diesen in Einzelkanäle 86. Die Rippen 84 versteifen zudem den Stutzen 36 und damit das Gehäuse. Die vordere Aufnahmenut 74 der Sonotrode 56, die bei dem Handstück 10 der Fign. 1 bis 6 vorhanden ist, kann entfallen, was fertigungs- und montagetechnisch (der vordere O-Ring und dessen Montage an der Sonotrode entfallen) vorteilhaft ist.

### Bezuaszeichenliste

- 10: (Einmalgebrauch-Phaco-Emulsifikations-)Handstück
- 10': (Einmalgebrauch-Phaco-Emulsifikations-)Handstück
- 12: Gehäuse
- 14: Außengehäuseteil
- 16: Innengehäuseteil
- 18: Ringraum
- 20: Außenvorsprünge
- 22: Außenvorsprünge
- 24: (hinteres) Ende des Gehäuses
- 26: Deckelteil
- 28: Durchführöffnung
- 30: Absaugschlauch
- 32: Anschlusskabel
- 34: (vorderes) Ende des Gehäuses
- 36: Stutzen
- 38: Austrittsöffnung für die Sonotrode am Außengehäuseteil
- 40: Ultraschallwandler
- 42: Halteelement
- 44: elastische Hülse (sleeve)
- 46: Werkzeug
- 48: Hohlnadel
- 49: Lumen der Hohlnadel
- 50: Einlassöffnung
- 52: Spülflüssigkeitsschlauch
- 54: Ringraum
- 55: Zwischenraum zwischen dem Stutzen und dem vorderen Ende der Sonotrode
- 56: Sonotrode
- 58: Resonator
- 60: Piezokeramikelement
- 62: Absaugkanal
- 64: vorderes Ende der Sonotrode
- 65: hinteres Ende der Sonotrode
- 66: Anschlussstutzen
- 68: Mittelabschnitt
- 70: Endstück
- 72: Gewindeeingriff zwischen Sonotrode und Resonator
- 74: Umfangsaufnahmenuten
- 76: Lagerring
- 78: Austrittsöffnung für die Sonotrode am Innengehäuseteil
- 80: Verstiftelung
- 82: Kühlöffnungen
- 84: (Führungs- und Versteifungs-)Rippen
- 86: Kanäle

## Patentansprüche

1. Einmalgebrauch-Phaco-Emulsifikationshandstück mit
- einem Gehäuse (12),
- einem Ultraschallwandler (40), der in dem Gehäuse (12) angeordnet ist, und
- einem Werkzeug (46), dass zwecks Ultraschallanregung mit dem Ult-raschallwandler (40) in Wirkverbindung steht,
- wobei der Ultraschallwandler (40) eine Sonotrode (56), einen Resonator (58) und ein zwischen diesen angeordnetes Piezokeramikelement (60) aufweist, und
- wobei die Sonotrode (56) und/oder der Resonator (58) eine Alumini-umlegierung aufweist oder aus einer Aluminiumlegierung besteht, deren Zugfestigkeit größer als 400 N/mm² ist.

2. Einmalgebrauch-Phaco-Emulsifikationshandstück nach Anspruch 1, **dadurch gekennzeichnet, dass** die Aluminiumlegierung eine hochfeste Aluminiumlegierung ist.

3. Einmalgebrauch-Phaco-Emulsifikationshandstück nach Anspruch 1 oder 2, **dadurch gekennzeichnet, dass** die Aluminiumlegierung eine Legierung ist aus der Gruppe der nachfolgend aufgeführten Aluminiumlegierungen: AICu4SiMg, AlCu4Mg1; AlZn4,5Mg1; AlZn5,5MgCu; AlZn5Mg3Cu; AIZn8MgCu.

4. Einmalgebrauch-Phaco-Emulsifikationshandstück nach einem der Ansprüche 1 bis 3, **dadurch gekennzeichnet, dass** die Sonotrode (56) einen benachbart zum Resonator (58) angeordneten Mittelabschnitt (68), in dem die Sonotrode (56) in dem Gehäuse (12) gelagert ist, ein mit dem Resonator (58) gekoppeltes hinteres Ende (24) und ein dem Resonator (58) abgewandtes, gegenüber dem Mittelabschnitt (68) verschlanktes vorderes Ende (34) aufweist, das in Wirkverbindung mit dem Werkzeug (46) steht, und dass sich durch die Sonotrode (56) von deren vorderen Ende (34) bis zu deren mit dem Resonator (58) verbundenen hinteren Ende (24) ein Absaugkanal (62) erstreckt.

5. Einmalgebrauch-Phaco-Emulsifikationshandstück nach Anspruch 4, **dadurch gekennzeichnet, dass** das vordere Ende (34) der Sonotrode (56) ein anderes metallisches Material als Aluminium oder als eine Aluminiumlegierung und insbesondere Titan oder Eisen oder Stahl oder eine eines dieser Metalle beinhaltende Legierung aufweist oder dass das vordere Ende (34) der Sonotrode (56) aus einem anderen metallischen Material als Aluminium oder als eine Aluminiumlegierung und insbesondere aus Titan oder Eisen oder Stahl oder einer eines dieser Metalle beinhaltende Legierung besteht.

6. Einmalgebrauch-Phaco-Emulsifikationshandstück nach einem der Ansprüche 1 bis 5, **dadurch gekennzeichnet, dass** der Resonator (58) hohlzylindrisch ausgeführt ist und dass die Sonotrode (56) ein mit dem Resonator (58) mechanisch gekoppeltes, sich durch den Resonator (58) erstreckendes hinteres Ende (24) aufweist, das zur Vorspannung des zwischen dem Resonator (58) und der Sonotrode (56) angeordneten Piezokeramikelements (60) in mechanischem Eingriff insbesondere in Gewindeeingriff (72) mit dem Resonator (58) steht.

7. Einmalgebrauch-Phaco-Emulsifikationshandstück nach einem der Ansprüche 1 bis 6, **dadurch gekennzeichnet, dass** die Sonotrode (56) an ihrem mit dem Werkzeug (46) in Wirkverbindung stehenden vorderen Ende (64) mit Spiel aus einer Öffnung des Gehäuses (12) herausgeführt ist und dass auf das Gehäuse (12) an dessen Öffnung ein Haltelement aufsetzbar, insbesondere aufsteck- oder aufschraubbar ist, von dem aus sich eine elastische Hülse (44) erstreckt, die das Werkzeug (46) von außen umgibt.

8. Einmalgebrauch-Phaco-Emulsifikationshandstück nach einem der Ansprüche 1 bis 7, **dadurch gekennzeichnet, dass** das Werkzeug (46) eine Hohlnadel (48) ist.

9. Einmalgebrauch-Phaco-Emulsifikationshandstück nach einem der Ansprüche 1 bis 8, **dadurch gekennzeichnet, dass** das Gehäuse (12) Kunststoff aufweist und/oder aus Kunststoff besteht.

10. Einmalgebrauch-Phaco-Emulsifikationshandstück nach Anspruch 7 oder nach Anspruch 7 und einem anderen der vorhergehenden Ansprüche, **dadurch gekennzeichnet, dass** das Gehäuse (12) ein zumindest einen Teil der Sonotrode (56) umgebendes Innengehäuseteil (16) und ein Außengehäuseteil (14) aufweist, zwischen denen sich ein Ringraum (18) für die Zuführung und/oder für die Weiterleitung von Spülflüssigkeit zum Werkzeug (46) befindet.

11. Einmalgebrauch-Phaco-Emulsifikationshandstück nach einem der Ansprüche 1 bis 10, **dadurch gekennzeichnet, dass** das Innengehäuseteil (16) Kühlöffnungen (82) aufweist, innerhalb derer die Sonotrode (56) und insbesondere der Mittelabschnitt (68) der Sonotrode (56) freiliegt und zur Kühlung mit Spülflüssigkeit in Kontakt gelangt.

12. Einmalgebrauch-Phaco-Emulsifikationshandstück nach Anspruch 9 oder 10, **dadurch gekennzeichnet, dass** das Innengehäuseteil (16) eine Austrittsöffnung (78) für das vordere Ende (34) der Sonotrode (56) aufweist und dass das Außengehäuseteil (14) eine mit der Austrittsöffnung (38) des Innengehäuseteils (16) fluchtende und von dieser in Längserstreckung der Sonotrode (56) beabstandete Austrittsöffnung (78) aufweist, in der das vordere Ende (34) der Sonotrode (56) angeordnet ist und/oder über die das vordere Ende (34) der Sonotrode (56) übersteht und auf die ein Halteelement (42) aufsetzbar, insbesondere aufsteck- oder aufschraubbar ist, von dem aus sich eine elastische Hülse (44) erstreckt, die das Werkzeug (46) von außen umgibt.

13. Einmalgebrauch-Phaco-Emulsifikationshandstück nach Anspruch 12, **dadurch gekennzeichnet, dass** sich an den Ringraum (18) zwischen dem Innengehäuseteil (16) und dem Außengehäuseteil (14) an der Austrittsöffnung (78) des Innengehäuseteils (16) ein weiterer Ringraum (54) anschließt, der von dem Außengehäuseteil (14) und dem vorderen Ende (64) der Sonotrode (56) gebildet ist und der sich bis in den durch ein Spiel zwischen dem vorderen Ende (34) der Sonotrode (56) und der Austrittsöffnung (38) des Außengehäuseteils (14) definierten Zwischenraum (55) erstreckt und von dort in einen Zwischenraum zwischen dem Werkzeug (46) und der elastischen Hülse (44) übergeht.

14. Einmalgebrauch-Phaco-Emulsifikationshandstück nach einem der Ansprüche 9 bis 13, **dadurch gekennzeichnet, dass** die Sonotrode (56) zwei axial beabstandete Aufnahmenuten (74) für jeweils einen Lagerring (76), insbesondere für einen elastischen Lagerring und vorzugsweise für einen O-Ring aufweist, wobei die beiden Lagerringe zur Lagerung des Ultraschallwandlers (40) von innen an dem Gehäuse (12) und insbesondere an dem Innengehäuseteil (16) anliegen.

15. Einmalgebrauch-Phaco-Emulsifikationshandstück nach einem der Ansprüche 9 bis 13, **dadurch gekennzeichnet, dass** die Sonotrode (56) an ihrem dem vorderen Ende (64) abgewandten hinteren Ende (65) oder in ihrem mittleren Abschnitt eine Aufnahmenut (74) für einen Lagerring (76), insbesondere für einen elastischen Lagerring und vorzugsweise für einen O-Ring, aufweist und dass das vordere Ende (64) der Sonotrode (56) durch nach innen vorstehende Rippen (84) o.dgl. Vorsprünge eines Stutzens (36) des Gehäuses (12) zentriert ist.

16. Einmalgebrauch-Phaco-Emulsifikationshandstück nach den Ansprüchen 11 und 15 oder nach einem der Ansprüche 9 bis 15, sofern auf Anspruch 11 rückbezogen, **dadurch gekennzeichnet, dass** die Kühlöffnungen (82) in dem zwischen den beiden Lagerringen liegenden Bereich des Gehäuses (12) bzw. des Innengehäuseteils (16) angeordnet sind und dass die beiden Aufnahmenuten (74) in dem Mittelabschnitt (68) der Sonotrode (56) ausgebildet sind.

17. Einmalgebrauch-Phaco-Emulsifikationshandstück nach einem der Ansprüche 9 bis 16, **dadurch gekennzeichnet, dass** das Gehäuse (12) eine mit dem Ringraum (18) in Fluidverbindung stehende Einlassöffnung (50) für die Zufuhr von Spülflüssigkeit aufweist.

18. Einmalgebrauch-Phaco-Emulsifikationshandstück nach einem der Ansprüche 9 bis 17, **dadurch gekennzeichnet, dass** das Gehäuse (12) an seinem dem vorderen Ende (34) der Sonotrode (56) abgewandten hinteren Ende (24) eine Durchführöffnung (28) für einen Absaugschlauch (30) aufweist, der mit dem sich durch die Sonotrode (56) hindurch erstreckenden Absaugkanal (62) verbunden ist.

19. Einmalgebrauch-Phaco-Emulsifikationshandstück nach einem der Ansprüche 9 bis 18, **dadurch gekennzeichnet, dass** aus dem Gehäuse (12) ein Anschlusskabel (32) herausgeführt ist, das an das Piezokeramikelement (60) angeschlossen ist.

## Claims

1. A single-use phacoemulsification handpiece, comprising
- a housing (12),
- an ultrasonic transducer (40) arranged in the housing (12), and
- a tool (46) which is operatively connected to the ultrasonic transducer (40) for the purpose of ultrasonic excitation,
- the ultrasonic transducer (40) comprising a sonotrode (56), a resonator (58) and a piezoceramic element (60) arranged therebetween, and
- the sonotrode (56) and/or the resonator (58) comprising an aluminum alloy or being made of an aluminum alloy, wherein the aluminum alloy has a tensile strength greater than 400 N/mm².

2. The single-use phacoemulsification handpiece according to claim 1, **characterized in that** the aluminum alloy is a high-strength aluminum alloy.

3. The single-use phacoemulsification handpiece according to claim 1 or 2, **characterized in that** the aluminum alloy is an alloy selected from the group of the following aluminum alloys: AICu4SiMg; AlCu4Mg1; ALZn4.5Mg1; AIZn5.5MgCu; AIZnMg3Cu; AlZn8MgCu.

4. The single-use phacoemulsification handpiece according to any one of claims 1 to 3, **characterized in that** the sonotrode (56) comprises a midsection (68) arranged adjacent to the resonator (58), in which the sonotrode (56) is supported in the housing (12), a rear end (24) coupled to the resonator (58) and a front end (34) which is arranged opposite the resonator (58) and is reduced compared to the midsection (68), the front end being operatively connected to the tool (46), and that a suctioning duct (62) extends through the sonotrode (56) from the front end (34) thereof to the rear end (24) thereof with the rear end (24) being connected to the resonator (58).

5. The single-use phacoemulsification handpiece according to claim 4, **characterized in that** the front end (34) of the sonotrode (56) comprises another metal material than aluminum or an aluminum alloy and in particular comprises titanium or iron or steel or an alloy comprising one of these metals, or that the front end (34) of the sonotrode (56) is made of another metal material than aluminum or an aluminum alloy and is made in particular of titanium or iron or steel or an alloy comprising one of these metals.

6. The single-use phacoemulsification handpiece according to any one of claims 1 to 5, **characterized in that** the resonator (58) is designed as a hollow cylinder and that the sonotrode (56) has a rear end (24) mechanically coupled to the resonator (58) and extending through the resonator (58), said rear end is in mechanical engagement, in particular in threaded engagement (72) with the resonator (58) for the purpose of biasing the piezoceramic element (60) arranged between the resonator (58) and the sonotrode (56).

7. The single-use phacoemulsification handpiece according to any one of claims 1 to 6, **characterized in that** the sonotrode (56) at its front end (64) operatively connected to the tool (46) extends through an opening of the housing (12) with a play, and that a holder element can be set, in particular plugged or screwed, on the housing (12) at its opening, wherein an elastic sleeve (44) extends from the holder element and surrounds the tool (46) at its outer side.

8. The single-use phacoemulsification handpiece according to any one of claims 1 to 7, **characterized in that** the tool (46) is a hollow needle (48).

9. The single-use phacoemulsification handpiece according to any one of claims 1 to 8, **characterized in that** the housing (12) comprises plastic material and/or is made of plastic material.

10. The single-use phacoemulsification handpiece according to claim 7 or according to claim 7 and any one of the preceding claims, **characterized in that** the housing (12) comprises an inner housing part (16) surrounding at least a portion of the sonotrode (56) and an outer housing part (14) between which an annular space (18) is provided for supplying and/or for directing rinsing fluid to the tool (46).

11. The single-use phacoemulsification handpiece according to any one of claims 1 to 10, **characterized in that** the inner housing part (16) comprises cooling openings (82) within which the sonotrode (56) and in particular the midsection (68) of the sonotrode (56) is exposed and comes in contact with rinsing fluid for the purpose of cooling.

12. The single-use phacoemulsification handpiece according to claim 9 or 10, **characterized in that** the inner housing part (16) comprises an exit opening (78) for the front end (34) of the sonotrode (56), and the outer housing part (14) comprises an exit opening (78) aligned with the exit opening (38) of the inner housing part (16) and spaced from the same in the longitudinal direction of the sonotrode (56), in which exit opening the front end (34) of the sonotrode (56) is arranged and/or beyond which the front end (34) of the sonotrode (56) protrudes and on which a holder element (42) can be set, in particular plugged or screwed, wherein an elastic sleeve (44) extends from the holder element and surrounds the tool (46) at its outer side.

13. The single-use phacoemulsification handpiece to claim 12, **characterized in that** the annular space (18) between the inner housing part (16) and the outer housing part (14) is adjoined at the exit opening (78) of the inner housing part (16) by a further annular space (54) which is formed by the outer housing part (14) and the front end (64) of the sonotrode (56) and which extends into an interspace (55) defined by a play between the front end (34) of the sonotrode (56) and the exit opening (38) of the outer housing part (14) and, from there, transitions into an interspace between the tool (46) and the elastic sleeve (44).

14. The single-use phacoemulsification handpiece according to any one of claims 9 to 13, **characterized in that** the sonotrode (56) comprises two axially spaced receiving grooves (74), each for one bearing ring (76), in particular for an elastic bearing ring and preferably an O-Ring, both bearing rings abutting on the housing (12) from inside and in particular on the inner housing part (16) for the purpose of supporting the ultrasonic transducer (40).

15. The single-use phacoemulsification handpiece according to any one of claims 9 to 13, **characterized in that** the sonotrode (56) comprises a receiving groove (74) for a bearing ring (76), in particular an elastic bearing ring and preferably an O-ring, at its rear end (65) opposite the front end (64) or in its central portion, and that the front end (64) of the sonotrode (56) is centered by inwardly protruding ribs (84) or the like protrusions of a connecting piece (36) of the housing (12).

16. The single-use phacoemulsification handpiece according to claims 11 and 15 or one of claims 9 to 15, if referred to claim 11, **characterized in that** the cooling openings (82) are arranged in the region of the housing (12) or the inner housing part (16) situated between the two bearing rings and the two receiving grooves (74) are formed in the midsection (68) of the sonotrode (56).

17. The single-use phacoemulsification handpiece according to any one of claims 9 to 16, **characterized in that** the housing (12) comprises an inlet opening (50) in fluid communication with the annular space (18) for the supply of rinsing fluid.

18. The single-use phacoemulsification handpiece according to any one of claims 9 to 17, **characterized in that** the housing (12) has a passage opening (28) for a suctioning hose (30) at its rear end (24) opposite the front end (34) of the sonotrode (56), which hose is connected to the suctioning duct (62) extending through the sonotrode (56).

19. The single-use phacoemulsification handpiece according to any one of claims 9 to 18, **characterized in that** a connection cable (32) connected to the piezoceramic element (60) extends led out from the housing (12).

## Revendications

1. Manche à usage unique pour la phacoémulsification, doté
- d'un boîtier (12),
- d'un transducteur à ultrasons (40), lequel est disposé dans le boîtier (12), et
- d'un outil (46), lequel est en liaison opérante avec le transducteur à ultrasons (40) à des fins d'excitation par ultrasons,
- dans lequel le transducteur à ultrasons (40) comporte une sonotrode (56), un résonateur (58) et un élément piézocéramique (60) disposé entre ceux-ci, et
- dans lequel la sonotrode (56) et/ou le résonateur (58) comporte un alliage d'aluminium ou est constitué(e) d'un alliage d'aluminium, dont la résistance en traction est supérieure à 400 N/mm².

2. Manche à usage unique pour la phacoémulsification selon la revendication 1, **caractérisé en ce que** l'alliage d'aluminium est un alliage d'aluminium à haute résistance.

3. Manche à usage unique pour la phacoémulsification selon la revendication 1 ou 2, **caractérisé en ce que** l'alliage d'aluminium est un alliage sélectionné parmi le groupe constitué par les alliages d'aluminium mentionnés ci-après : AlCu4SiMg ; AlCu4Mg1 ; AlZn4,5Mg1 ; AlZn5,5MgCu ; AlZn5Mg3Cu ; AlZn8MgCu.

4. Manche à usage unique pour la phacoémulsification selon l'une des revendications 1 à 3, **caractérisé en ce que** la sonotrode (56) comporte une section centrale (68) disposée de manière adjacente au résonateur (58), la sonotrode (56) étant montée dans le boîtier (12) dans cette section, une extrémité arrière (24) couplée au résonateur (58) et une extrémité avant (34) plus mince que la section centrale (68), opposée au résonateur (58) et en liaison opérante avec l'outil (46), et **en ce qu'**un canal d'aspiration (62) s'étend à travers la sonotrode (56) depuis son extrémité avant (34) jusqu'à son extrémité arrière (24) reliée au résonateur (58).

5. Manche à usage unique pour la phacoémulsification selon la revendication 4, **caractérisé en ce que** l'extrémité avant (34) de la sonotrode (56) comporte un matériau métallique autre que l'aluminium ou qu'un alliage d'aluminium et en particulier du titane ou du fer ou de l'acier ou un alliage contenant l'un de ces métaux, ou **en ce que** l'extrémité avant (34) de la sonotrode (56) est constituée d'un matériau métallique autre que l'aluminium ou qu'un alliage d'aluminium et en particulier de titane ou de fer ou d'acier ou d'un alliage contenant l'un de ces métaux.

6. Manche à usage unique pour la phacoémulsification selon l'une des revendications 1 à 5, **caractérisé en ce que** le résonateur (58) est configuré comme cylindre creux et **en ce que** la sonotrode (56) comporte une extrémité arrière (24) couplée mécaniquement au résonateur (58) et s'étendant à travers le résonateur (58), laquelle s'engage mécaniquement, en particulier en engagement fileté (72), avec le résonateur (58) pour solliciter l'élément piézocéramique (60) disposé entre le résonateur (58) et la sonotrode (56).

7. Manche à usage unique pour la phacoémulsification selon l'une des revendications 1 à 6, **caractérisé en ce que** la sonotrode (56) sort, à son extrémité avant (64) en liaison opérante avec l'outil (46), avec du jeu hors d'une ouverture du boîtier (12) et **en ce que** sur le boîtier (12), à son ouverture, peut être placé, en particulier enfiché ou vissé, un élément de maintien à partir duquel s'étend un manchon élastique (44) qui entoure l'outil (46) de l'extérieur.

8. Manche à usage unique pour la phacoémulsification selon l'une des revendications 1 à 7, **caractérisé en ce que** l'outil (46) est une aiguille creuse (48).

9. Manche à usage unique pour la phacoémulsification selon l'une des revendications 1 à 8, **caractérisé en ce que** le boîtier (12) comporte du plastique et/ou est constitué de plastique.

10. Manche à usage unique pour la phacoémulsification selon la revendication 7 ou selon la revendication 7 et une autre des revendications précédentes, **caractérisé en ce que** le boîtier (12) comporte une partie intérieure de boîtier (16) entourant au moins une partie de la sonotrode (56) et une partie extérieure de boîtier (14), entre lesquelles se trouve un espace annulaire (18) pour alimenter et/ou conduire du liquide de rinçage vers l'outil (46).

11. Manche à usage unique pour la phacoémulsification selon l'une des revendications 1 à 10, **caractérisé en ce que** la partie intérieure du boîtier (16) comporte des ouvertures de refroidissement (82) à l'intérieur desquelles la sonotrode (56) et en particulier la section centrale (68) de la sonotrode (56) est exposée et entre en contact avec un liquide de rinçage pour le refroidissement.

12. Manche à usage unique pour la phacoémulsification selon la revendication 9 ou 10, **caractérisé en ce que** la partie intérieure de boîtier (16) comporte une ouverture de sortie (78) pour l'extrémité avant (34) de la sonotrode (56) et **en ce que** la partie extérieure de boîtier (14) comporte une ouverture de sortie (38) alignée avec l'ouverture de sortie (78) de la partie intérieure de boîtier (16) et espacée par rapport à celle-ci dans l'étendue longitudinale de la sonotrode (56), à l'intérieur de laquelle l'extrémité avant (34) de la sonotrode (56) est disposée et/ou au-dessus de laquelle l'extrémité avant (34) de la sonotrode (56) fait saillie et sur laquelle un élément de maintien (42) peut être placé, en particulier enfiché ou vissé, à partir duquel s'étend un manchon élastique (44) qui entoure l'outil (46) de l'extérieur.

13. Manche à usage unique pour la phacoémulsification selon la revendication 12, **caractérisé en ce qu'**un espace annulaire ultérieur (54) se raccorde à l'espace annulaire (18) entre la partie intérieure de boîtier (16) et la partie extérieure de boîtier (14) à l'ouverture de sortie (78) de la partie intérieure de boîtier (16), lequel est formé par la partie extérieure de boîtier (14) et l'extrémité avant (64) de la sonotrode (56) et s'étend dans l'espace intermédiaire (55) défini par un jeu entre l'extrémité avant (34) de la sonotrode (56) et l'ouverture de sortie (38) de la partie extérieure de boîtier (14) et, à partir de là, se transforme en un espace intermédiaire entre l'outil (46) et le manchon élastique (44).

14. Manche à usage unique pour la phacoémulsification selon l'une des revendications 9 à 13, **caractérisé en ce que** la sonotrode (56) comporte deux rainures de logement (74) axialement espacées, chacune pour une bague de palier (76), en particulier pour une bague de palier élastique et de préférence pour un joint torique, dans lequel les deux bagues de palier appuient de l'intérieur sur le boîtier (12) et en particulier sur la partie intérieure du boîtier (16) pour le montage du transducteur ultrasonore (40).

15. Manche à usage unique pour la phacoémulsification selon l'une des revendications 9 à 13, **caractérisé en ce que** la sonotrode (56) comporte, à son extrémité arrière (65) se détournant de l'extrémité avant (64) ou dans sa partie centrale, une rainure de logement (74) pour une bague de palier (76), en particulier pour une bague de palier élastique et de préférence pour un joint torique, et **en ce que** l'extrémité avant (64) de la sonotrode (56) est centrée par des nervures (84) ou des saillies analogues saillant vers l'intérieur d'un embout (36) du boîtier (12).

16. Manche à usage unique pour la phacoémulsification selon les revendications 11 et 15, ou selon l'une des revendications 9 à 15, tant que celle-ci renvoie à la revendication 11, **caractérisé en ce que** les ouvertures de refroidissement (82) sont disposées dans la zone du boîtier (12) ou de la partie intérieure de boîtier (16) située entre les deux bagues de palier et **en ce que** les deux rainures de logement (74) sont réalisés dans la section centrale (68) de la sonotrode (56).

17. Manche à usage unique pour la phacoémulsification selon l'une des revendications 9 à 16, **caractérisé en ce que** le boîtier (12) comporte une ouverture d'entrée (50) en liaison fluidique avec l'espace annulaire (18) pour l'alimentation en liquide de rinçage.

18. Manche à usage unique pour la phacoémulsification selon l'une des revendications 9 à 17, **caractérisé en ce que** le boîtier (12) comporte, à son extrémité arrière (24) opposée à l'extrémité avant (34) de la sonotrode (56), une ouverture de passage (28) pour un tuyau d'aspiration (30) relié au canal d'aspiration (62) s'étendant à travers la sonotrode (56).

19. Manche à usage unique pour la phacoémulsification selon l'une des revendications 9 à 18, **caractérisé en ce qu'**un câble de raccordement (32) sort du boîtier (12) et est raccordé à l'élément piézocéramique (60).
